# EUROPEAN PATENT APPLICATION

(11) **EP 4 481 746 A1**
(43) Date of publication of application: **25.12.2024**
(21) Application number: 23756665.8
(22) Date of filing: 17.02.2023
(51) Int. Cl.: G16B 20/20, G16B 30/10, G16B 50/00, G16B 40/00, G16H 50/50, G16H 50/20

(54) **GENETIC VARIATION ANALYSIS METHOD BASED ON NUCLEIC ACID SEQUENCING**

(30) Priority: 17.02.2022 KR 20220020483
(71) Applicant: SCL Healthcare, Co., Ltd., Yongin-si Gyeonggi-do 16954 (KR)
(72) Inventor: LEE, Sanghoo, Yongin-si Gyeonggi-do 16954 (KR); HONG, Jeong Hoon, Yongin-si Gyeonggi-do 16954 (KR); LEE, Mi-Kyeong, Yongin-si Gyeonggi-do 16954 (KR); BAIK, Saeyun, Yongin-si Gyeonggi-do 16954 (KR); LEE, Kyoung-Ryul, Yongin-si Gyeonggi-do 16954 (KR)
(74) Representative: Mewburn Ellis LLP
(86) International application number: PCT/KR2023/002310
(87) International publication number: WO 2023/158253

(57) **Abstract**

The types of genetic variants detected by NGS are very wide and not all genetic variants always lead to diseases, and thus it is difficult to quickly and accurately interpret the meaning of disease relevance for detected genetic variants. The present invention relates to a method of interpreting genetic variants based on nucleic acid sequencing. The method of interpreting genetic variants according to the present invention provides a logic tree for interpreting NGS variant data, which can classify the pathogenicity of genetic variants based on the ACMG guidelines and determine the level of pathogenicity of the genetic variants, and thus it is expected to be widely used in the life sciences and medical health fields.

## Description

### Technical Field

The present invention relates to a method of interpreting genetic variants based on nucleic acid sequencing.

### Background Art

With the rapid development of next-generation sequencing (NGS), a high-throughput sequencing technique, studies on genomic data, including tracking variants, have been actively conducted, and continued efforts have been made to use NGS for disease diagnosis. However, since the types of genetic variants detected by NGS are very wide and there are also genetic variants that only cause simple phenotypic differences, not all genetic variants always lead to diseases. Thus, it is difficult to quickly and accurately interpret the meaning of disease relevance for detected genetic variants. In addition, there has emerged the need for a technology capable of conveying the meaning of genetic variants, interpreted from NGS information, in common terms through smooth communication between scientists and medical professionals around the world. In this context, the American Medical College of Medical Genetics and Genomics (ACMG), the Association for Molecular Pathology (AMP), and the College of American Pathologists (CAP) jointly established the ACMG guidelines that recommend classifying genetic variants into five pathogenicity classes based on a total of 28 criteria. However, it is still very complicate to integrate information on various genetic variants detected by NGS and determine the pathogenicity of variants according to the ACMG guidelines, and these processes are difficult to apply to research and clinical practice.

Therefore, the present invention has been made in order to solve the above-described problems and relates to a method of interpreting genetic variants based on nucleic acid sequencing. The method of interpreting genetic variants according to the present invention provides a logic tree for interpreting NGS variant data, which can classify the pathogenicity of genetic variants based on the ACMG guidelines and determine the level of pathogenicity of the genetic variants, and thus it is expected to be widely used in the life sciences and medical health fields.

### DISCLOSURE

### Technical Problem

The present invention has been made in order to solve the above-described problems occurring in the prior art, and relates to a method of interpreting genetic variants based on nucleic acid sequencing.

In one aspect, the present invention provides a method for determining the pathogenicity of genetic variants.

In another aspect, the present invention provides an apparatus for determining the pathogenicity of genetic variants.

In still another aspect, the present invention provides a method for predicting disease occurrence in a subject.

In yet another aspect, the present invention provides a method of providing information for diagnosis of the cause of disease in a subject.

However, objects to be achieved by the present invention are not limited to the objects mentioned above, and other objects not mentioned about may be clearly understood by those skilled in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present invention pertains.

Throughout the present specification, it is to be understood that when any part is referred to as "comprising" any component, it does not exclude other components, but may further comprise other components, unless otherwise specified.

The present invention provides a logic tree for interpreting NGS variant data, which can classify the pathogenicity of genetic variants based on the ACMG guidelines, established jointly by the American Medical College of Medical Genetics and Genomics (ACMG) and the Association for Molecular Pathology, and determine the level of pathogenicity of the genetic variants.

In the present invention, the "ACMG guidelines" refers to guidelines for the interpretation of sequence variants, established jointly by the American Medical College of Medical Genetics and Genomics (ACMG), the Association for Molecular Pathology (AMP), and the College of American Pathologists (CAP). The ACMG guidelines provide a classification method of classifying genetic variants into five pathogenicity classes based on a total of 28 criteria.

Table 1 below shows the method of classifying the pathogenicity of genetic variants according to the ACMG guidelines, and Table 2 below shows the method of determining pathogenicity.

**[Table 1]**

| **Pathogenicity (classification)** | **Pathogenicity (sub-classification)** | **Criteria for classification** |
|---|---|---|
| Pathogenic criteria | Very strong | PVS1-LOF (loss-of-function) |
| | Strong | PS1-same aa change known |
| | | PS2-de novo |
| | | PS3-functional test |
| | | PS4-affected individuals |
| | Moderate | PM1-mutation hotspot |
| | | PM2-absent from controls |
| | | PM3-cis/trans testing |
| | | PM4-change in protein length |
| | | PM5-other aa change in same codon |
| | | PM6-de novo |
| | Supporting | PP1-cosegregation |
| | | PP2-rare benign missense |
| | | PP3-in silico evidence |
| | | PP4-phenotype match |
| | | PP5-source without evidence |
| Benign criteria | Stand-alone | BA1-MAF > 5% |
| | Strong | BS1-MAF > prevalence |
| | | BS2-in healthy individuals |
| | | BS3-functional test |
| | | BS4-lack of cosegregation |
| | Supporting | BP1-missense in LOF gene |
| | | BP2-cis/trans testing |
| | | BP3-repetitive region |
| | | BP4-in silico evidence |
| | | BP5-other known case of disease |
| | | BP6-source without evidence |
| | | BP7-synonymous without splicing effect |

**[Table 2]**

| | | |
|---|---|---|
| Pathogenic | (i) 1 Very strong (PVS1) *AND* | |
| | | (a) ≥ 1 Strong (PS1-PS4) *OR* |
| | | (b) ≥ 2 Moderate (PM1-PM6) *OR* |
| | | (c) 1 Moderate (PM1-PM6) and 1 Supporting (PP1-PP5) *OR* |
| | | (d) ≥ 2 Supporting (PP1-PP5) |
| | (ii) ≥ 2 Strong (PS1-PS4) *OR* | |
| | (iii) 1 Strong (PS1-PS4)*AND* | |
| | | (a) ≥ 3 Moderate (PM1-PM6) *OR* |
| | | (b) 2 Moderate (PM1-PM6) and ≥ 2 Supporting (PP1-PP5) *OR* |
| | | (c) 1 Moderate (PM1-PM6) and ≥ 4 Supporting (PP1-PP5) |
| Likely pathogenic | (i) 1 Very strong (PVS1) and 1 Moderate (PM1-PM6) *OR* | |
| | (ii) 1 Strong (PS1-PS4) and 1-2 Moderate (PM1-PM6) *OR* | |
| | (iii) 1 Strong (PS1-PS4) and ≥ 2 Supporting (PP1-PP5) *OR* | |
| | (iv) ≥ 3 Moderate (PM1-PM6) *OR* | |
| | (v) 2 Moderate (PM1-PM6) and ≥ 2 Supporting (PP1-PP5) *OR* | |
| | (vi) 1 Moderate (PM1-PM6) and ≥ 4 Supporting (PP1-PP5) | |
| Benign | (i) 1 Stand-alone (BA1) *OR* | |
| | (ii) ≥ 2 Strong (BS1-BS4) | |
| Likely benign | (i) 1 Strong (BS1-BS4) and 1 Supporting (BP1-BP7) *OR* | |
| | (ii) ≥ 2 Supporting (BP1-BP7) | |
| Uncertain significance | (i) other criteria shown above are not met *OR* | |
| | (ii) the criteria for benign and pathogenic are contradictory | |

Additional specific information regarding the ACMG guidelines including Tables 1 and 2 above can be found in the prior art document (S. Richards et al., Genet Med. 2015 May; 17(5): 405-424.), etc. known in the art, which may be applied to the present invention.

In the present invention, the term "logic tree", "logic tree system", or "system" refers to processes that are to be performed by computer programming, etc. to solve a given task. When the desired result can be obtained by mechanical processing according to a certain order, the certain order is called a logic tree for the purpose. It may also be replaced with the term "algorithm".

In the present invention, the logic tree system is an algorithm including a method for classifying the pathogenicity of genetic variants based on the ACMG guidelines from genetic variant information detected by NGS and determining the level of pathogenicity of the genetic variants, or a system for implementing the algorithm.

The algorithm of the present invention retrieves various data, which help interpret genetic variants, from various databases known in the art, extracts necessary information, obtains classification criteria according to the ACMG guidelines, and determines the level of pathogenicity of the genetic variants based on the criteria.

The algorithm of the present invention is characterized by extracting and using only some of the necessary information without processing the information retrieved from various databases known in the art.

In addition, the algorithm of the present invention is characterized by using different databases (DBs) depending on the retrieved information of interest. The retrieved information of interest may be clinical characteristic information, popular single-nucleotide polymorphism (SNP) frequency information, repeat sequence information, protein domain information, and/or in-silico prediction information, wherein the in-silico prediction information may be missense prediction information, splice prediction information, and/or conservation prediction information. In this case, preferably, regarding the databases from which each of the information is retrieved, the clinical characteristic information may be retrieved from a database based on clinical characteristic information provided by the U.S. National Center for Biotechnology Information (NCBI), the popular SNP frequency information may be retrieved from a database that provides SNP frequency information for an unspecified number of people (10,000 or more), the repeat sequence information may be retrieved from a database containing interspersed repeats and low-complexity DNA sequences, the protein domain information may be retrieved from a database that is a collection of protein families, each represented by multiple sequence alignments and hidden Markov models, and the in-silico prediction information may be retrieved from a database divided into missense prediction information, splice prediction information, and conservation prediction information. For example, the clinical characteristic information may be retrieved from the ClinVar database, the popular SNP frequency information may be retrieved from the GnomAD database, the repeat sequence information may be retrieved from the RepeatMasker database, the protein domain information may be retrieved from the Pfam database, the missense prediction information may be retrieved from a database that uses the MetaSVM, REVEL, Eigen, Polyphen2, Provean, or VEST3 algorithm, the splice prediction information may be retrieved from a database that uses the Ada_score & Rf_score algorithm, and the conservation prediction information may be retrieved from a database that uses the GERP++ algorithm, without being limited thereto. The sources of the above databases are shown in Tables 3 and 4 below.

**[Table 3]**

| | **Remarks** | **Example of DB** |
|---|---|---|
| Clinical characteristic information | Database based on clinical characteristic information provided by the U.S. National Center for Biotechnology Information (NCBI) | ClinVar |
| Popular SNP frequency information | Database that provides SNP frequency information for an unspecified number of people (125,748 people) | GnomAD |
| Repeat sequence information | Database containing interspersed repeats and low-complexity DNA sequences | RepeatMasker |
| Protein domain information | Database that is a collection of protein families, each represented by multiple sequence alignments and hidden Markov models | Pfam |
| In-silico prediction information | Estimating using an algorithm to predict pathogenicity | Table 4 |

**[Table 4]**

| | **Algorithm** | **Source** |
|---|---|---|
| Missense prediction information | MetaSVM | Meta-analytic support vector machine for integrating multiple omics data. BioData Min. 2017 |
| | REVEL | REVEL: An ensemble method for predicting the pathogenicity of rare missense variants. Am J Hum Genet. 2016 |
| | Eigen | A spectral approach integrating functional genomic annotations for coding and noncoding variants. Nat Genet. 2016 |
| | Polyphen2 | Predicting functional effect of human missense mutations using PolyPhen-2. Curr Protoc Hum Genet. 2013 |
| | Provean | Predicting the functional effect of amino acid substitutions and Indels. PlosOne 2012 |
| | VEST3 | Identifying mendelian disease genes with the variant effect scoring tool. BMC Genomics. 2013 |
| Splice prediction information | Ada_score & Rf_score | In silico prediction of splice-altering single nucleotide variants in the human genome. Nucleic Acids Res. 2014 |
| Conservation prediction information | GERP++ | Identifying a high fraction of the human genome to be under selective constraint using GERP++. PLOS Computational Biology. 2010 |

One embodiment of the present invention provides a method for determining pathogenicity of genetic variants, comprising steps of: (a) obtaining information on genetic variants from sequencing results; (b) classifying the pathogenicity of the genetic variants by comparing the information on genetic variants with clinical characteristic information, single-nucleotide polymorphism frequency information, repeat sequence information, protein domain information, and in-silico prediction information, and (c) determining the level of pathogenicity of the genetic variants. The sequencing may be conventional Sanger-based dideoxy sequencing, or new massively parallel sequencing such as next-generation sequencing, without being limited thereto. In the method for determining pathogenicity of genetic variants, the clinical characteristic information, single-nucleotide polymorphism frequency information, repeat sequence information, protein domain information, and in-silico prediction information are extracted from public databases. In the method for determining pathogenicity of genetic variants, the clinical characteristic information is extracted from a database based on clinical characteristic information provided by the U.S. National Center for Biotechnology Information (NCBI), the single-nucleotide polymorphism frequency information is extracted from a database that provides single-nucleotide polymorphism (SNP) frequency information for an unspecified number of people, the repeat sequence information is extracted from a database containing interspersed repeats and low-complexity DNA sequences, the protein domain information is extracted from a database that is a collection of protein families, each represented by multiple sequence alignments and hidden Markov models, and the in-silico prediction information is extracted from an in-silico database divided into missense prediction information, splice prediction information, and conservation prediction information. In addition, in the method for determining pathogenicity of genetic variants, step (b) of classifying the pathogenicity of the genetic variants comprises classifying the pathogenicity as "very strong", "strong", "moderate", or "supporting" for pathogenic criteria, and classifying the pathogenicity as "stand-alone", "strong", or "supporting" for benign criteria, or step (b) of classifying the pathogenicity of the genetic variants comprises classifying the pathogenicity as "very strong" stage 1, "strong" stage 1 to 4, "moderate" stage 1 to 6, or "supporting" stage 1 to 5 for pathogenic criteria, and classifying the pathogenicity as "stand-alone" stage 1, "strong" stage 1 to 4, or "supporting" stage 1 to 7 for benign criteria.

In the method for determining pathogenicity of genetic variants according to the present invention, the clinical characteristic information is applied to any one or more classes selected from the group consisting of very "strong" stage 1 for pathogenic criteria, "strong" stage 1 for pathogenic criteria, "moderate" stage 1 for pathogenic criteria, "moderate" stage 5 for pathogenic criteria, "supporting" stage 2 for pathogenic criteria, "strong" stage 1 for benign criteria, and "supporting" stage 6 for benign criteria, and the single-nucleotide polymorphism frequency information is applied to any one or more classes selected from the group consisting of "moderate" stage 2 for pathogenic criteria, "stand-alone" stage 1 for benign criteria, "supporting" stage 1 for benign criteria, and "supporting" stage 2 for benign criteria. In the method for determining pathogenicity of genetic variants, the repeat sequence information is applied to any one or more classes selected from the group consisting of "moderate" stage 4 for pathogenic criteria, and "supporting" stage 3 for benign criteria. In the method for determining pathogenicity of genetic variants, the protein domain information is applied to the class of "moderate" stage 1 for pathogenic criteria. In the method for determining pathogenicity of genetic variants, the in-silico prediction information is applied to any one or more classes selected from the group consisting of "supporting" stage 3 for pathogenic criteria, "supporting" stage 4 for benign criteria, and "supporting" stage 7 for benign criteria. Specifically, in the method for determining pathogenicity of genetic variants, the clinical characteristic information is applied to class PVS1, PS1, PM1, PM5, PP2, BS1, BP1, or BP6. In the method for determining pathogenicity of genetic variants, the single-nucleotide polymorphism frequency information is applied to class PM2, BA1, BS1, or BS2. In the method for determining pathogenicity of genetic variants, the repeat sequence information is applied to class PM4 or BP3. In the method for determining pathogenicity of genetic variants, the protein domain information is applied to class PM1. In the method for determining pathogenicity of genetic variants, the in-silico prediction information is applied to class PP3, BP4, or BP7. However, the present invention is not limited thereto.

In addition, in the method for determining pathogenicity of genetic variants according to the present invention, step (c) of determining the level of pathogenicity comprises classifying the level of pathogenicity as pathogenic, likely pathogenic, benign, likely benign, or uncertain significance. In the method for determining pathogenicity of genetic variants, the likely benign is classified into uncertain significance, uncertain significance-pathogenic, and uncertain significance-benign. In the method for determining pathogenicity of genetic variants, the determining of the level of pathogenicity is performed according to the classification shown in Table 7 in the present specification.

Another embodiment of the present invention provides an apparatus for determining pathogenicity of genetic variants, comprising: (a) an input unit configured to input information on genetic variants obtained from sequencing results; (b) a classification unit configured to classify the pathogenicity of genetic variants by comparing the information on genetic variants with clinical characteristic information, single-nucleotide polymorphism frequency information, repeat sequence information, protein domain information, and in-silico prediction information; and (c) a determination unit configured to determine the level of pathogenicity of the genetic variants. Details regarding each unit of the apparatus overlap with those described above with respect to the method for determining the pathogenicity of genetic variants, and thus will be omitted below to avoid excessive complexity of the present specification.

Still another embodiment of the present invention provides a method for predicting disease occurrence in a subject, comprising steps of: (a) performing sequencing on a sample isolated from a subject of interest; (b) determining pathogenicity of genetic variants according to the method of claim 1; and (c) predicting disease occurrence in the subject based on the result of determining the pathogenicity. Details regarding each step of the method for predicting disease occurrence overlap with those described above with respect to the method for determining the pathogenicity of genetic variants, and thus will be omitted below to avoid excessive complexity of the present specification.

Yet another embodiment of the present invention provides a method of providing information for diagnosis of the cause of disease in a subject, comprising steps of: (a) performing sequencing on a sample isolated from a subject of interest; (b) determining pathogenicity of genetic variants according to the method of claim 1; and (c) determining the cause of disease in the subject based on the result of determining the pathogenicity. Details regarding each step of the method of providing information for diagnosis of the cause of disease in a subject overlap with those described above with respect to the method for determining the pathogenicity of genetic variants, and thus will be omitted below to avoid excessive complexity of the present specification.

Hereinafter, the present invention will be described in detail based on examples.

### Advantageous Effects

The method of interpreting genetic variants according to the present invention provides a logic tree for interpreting NGS variant data, which can classify the pathogenicity of genetic variants based on the ACMG guidelines and determine the level of pathogenicity of the genetic variants, and thus it is expected to be widely used in the life sciences and medical health fields.

### Brief Description of Drawings

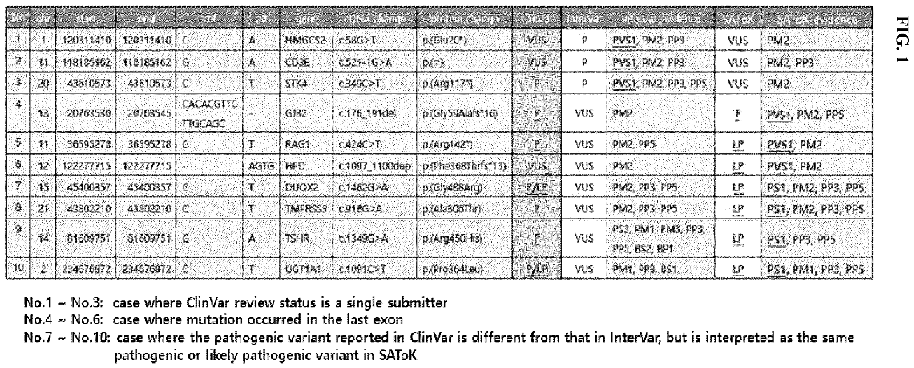

FIG. 1 shows the results of comparing the accuracy of determining the level of pathogenicity between a logic tree of the present invention and a control logic tree, according to an example of the present invention.

### Best Mode

In the best mode, the present invention provides a method for determining pathogenicity of genetic variants, comprising steps of: (a) obtaining information on genetic variants from sequencing results; (b) classifying pathogenicity of the genetic variants by comparing the information on genetic variants with clinical characteristic information, single-nucleotide polymorphism frequency information, repeat sequence information, protein domain information, and in-silico prediction information, and (c) determining the level of pathogenicity of the genetic variants. In the method for determining pathogenicity of genetic variants, step (b) of classifying pathogenicity of the genetic variants comprises classifying the pathogenicity as "very strong" stage 1, "strong" stage 1 to 4, "moderate" stage 1 to 6, or "supporting" stage 1 to 5 for pathogenic criteria, and classifying comprises the pathogenicity as "stand-alone" stage 1, "strong" stage 1 to 4, or "supporting" stage 1 to 7 for benign criteria, wherein the clinical characteristic information is applied to any one or more classes selected from the group consisting of very "strong" stage 1 for pathogenic criteria, "strong" stage 1 for pathogenic criteria, "moderate" stage 1 for pathogenic criteria, "moderate" stage 5 for pathogenic criteria, "supporting" stage 2 for pathogenic criteria, "strong" stage 1 for benign criteria, and "supporting" stage 6 for benign criteria. In addition, step (c) of determining the level of pathogenicity comprises classifying the level of pathogenicity as "pathogenic", "likely pathogenic", "benign", "likely benign", "uncertain significance", "uncertain significance-pathogenic", or "uncertain significance-benign".

### Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

Throughout the present specification, it is to be understood that when any part is referred to as "comprising" any component, it does not exclude other components, but may further comprise other components, unless otherwise specified.

### Example 1. Development of Logic Tree for Interpreting NGS Variant Data

The present inventors have developed a logic tree for interpreting NGS variant data, which can classify the pathogenicity of genetic variants based on the ACMG guidelines and determine the level of pathogenicity of the genetic variants.

Hereinafter, the algorithm of the present invention will be referred to as "SATok".

Typically, the output result changes infinitely depending on the input value to the algorithm. Thus, for the purpose of the present invention, selection of input information is very important for rapid and accurate interpretation of NGS variant data. For the logic tree of the present invention, as input information, clinical characteristic information, popular single-nucleotide polymorphism (SNP) frequency information, repeat sequence information, protein domain information, and in-silico prediction information were selected, and as the in-silico prediction information, missense prediction information, splice prediction information, and conservation prediction information were selected. Specifically, the clinical characteristic information was information retrieved from a database based on clinical characteristic information provided by the U.S. National Center for Biotechnology Information (NCBI), the popular SNP frequency information was information retrieved from a database that provides SNP frequency information for an unspecified number of people (10,000 or more people), the repeat sequence information was information retrieved from a database containing interspersed repeats and low-complexity DNA sequences, and the protein domain information was information retrieved from a database that is a collection of protein families, each represented by multiple sequence alignments and hidden Markov models. In addition, as the in-silico prediction information, missense prediction information, splice prediction information, and conservation prediction information were separately retrieved.

The retrieved information was applied to the method of classifying the pathogenicity of genetic variants according to the ACMG guidelines, but the retrieved information applied was different between the pathogenicity classifications of the ACMG guidelines. The results of the application are shown in Tables 5 and 6 below. In Tables 5 and 6 below, "information 1" indicates clinical characteristic information retrieved from a database based on clinical characteristic information provided by the U.S. National Center for Biotechnology Information (NCBI), "information 2" indicates popular SNP frequency information retrieved from a database that provides SNP frequency information for an unspecified number of people (100 or more people), "information 3" indicates repeat sequence information retrieved from a database containing interspersed repeats and low-complexity DNA sequences, "information 4" indicates protein domain information retrieved from a database that is a collection of protein families, each represented by multiple sequence alignments and hidden Markov models, and "information 5" indicates retrieved in-silico prediction information divided into missense prediction information, splice prediction information, and conservation prediction information. In addition, the mark "O" indicates the case where the information was applied, the mark "X" indicates the case where the information was not applied, and "-" indicates the case where the information was not automatically classified by the logic tree of the present invention.

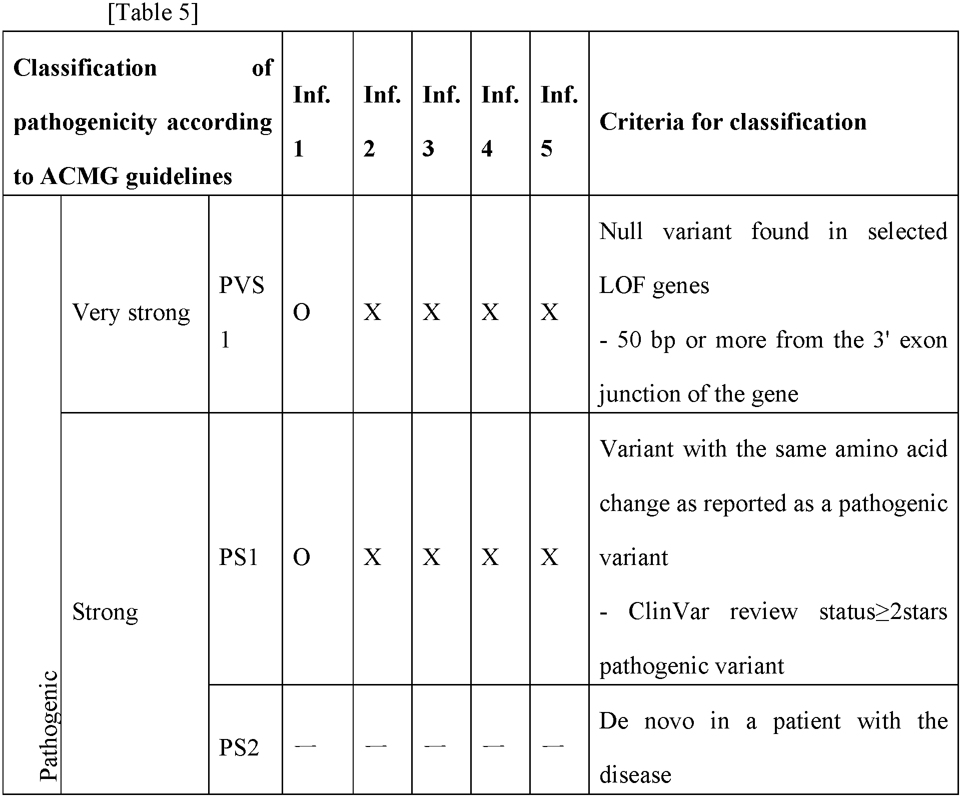

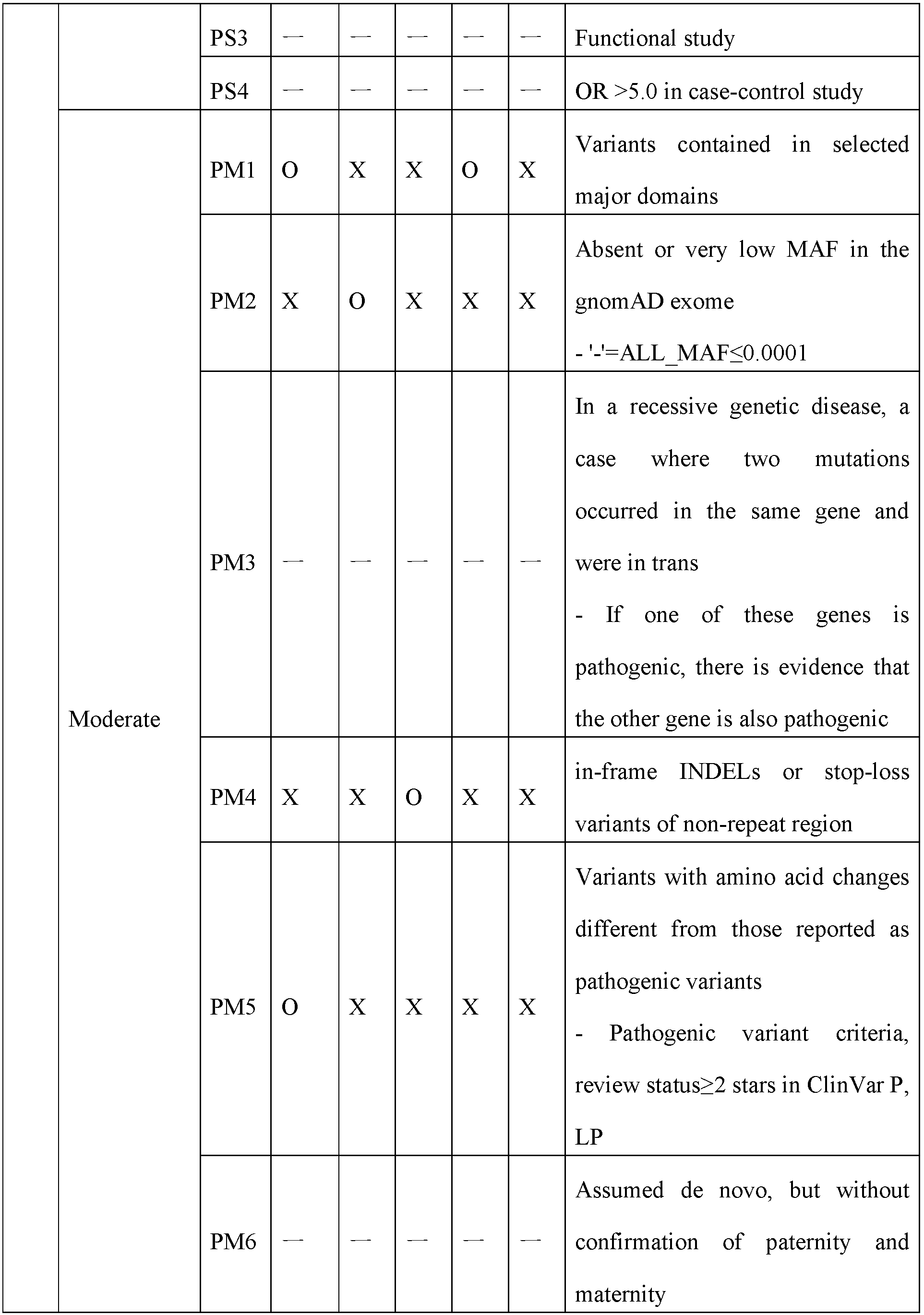

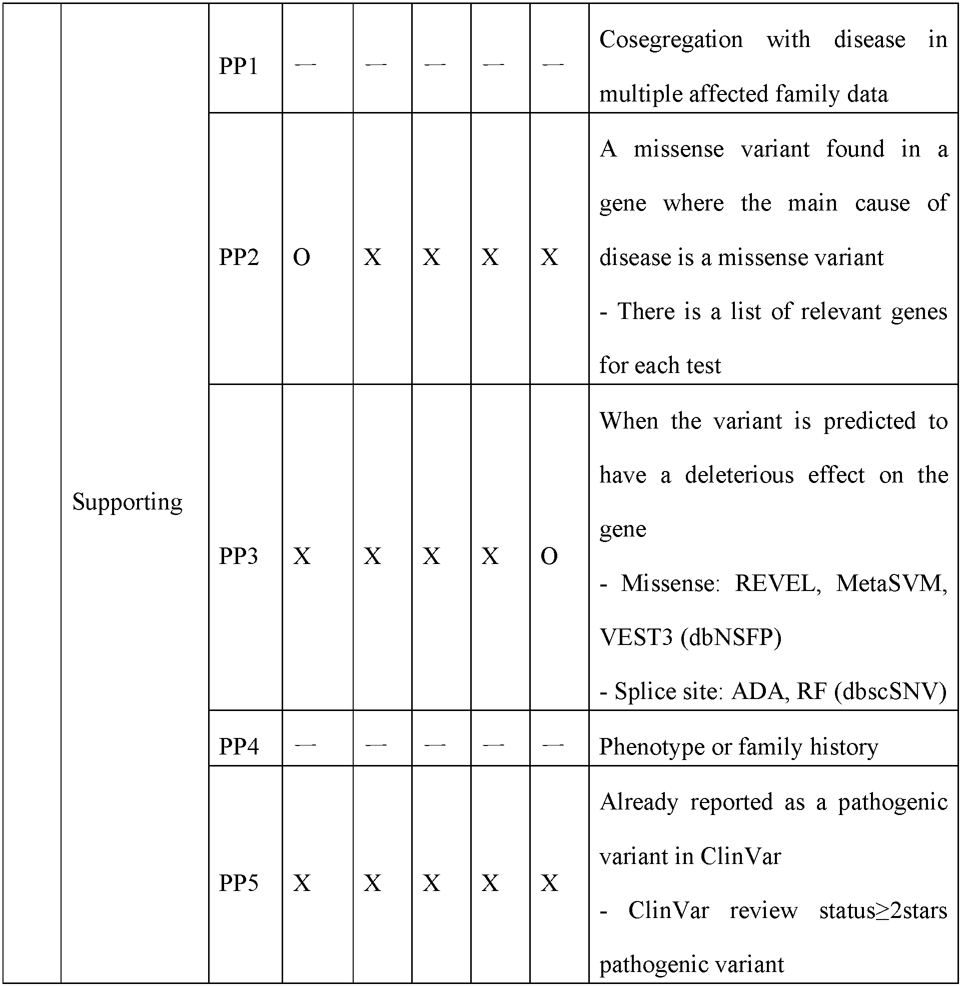

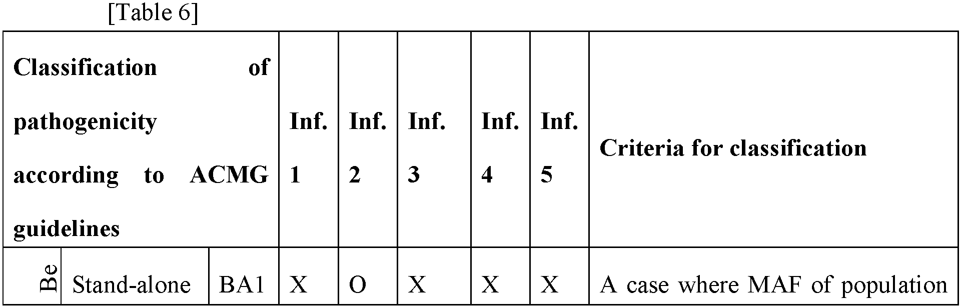

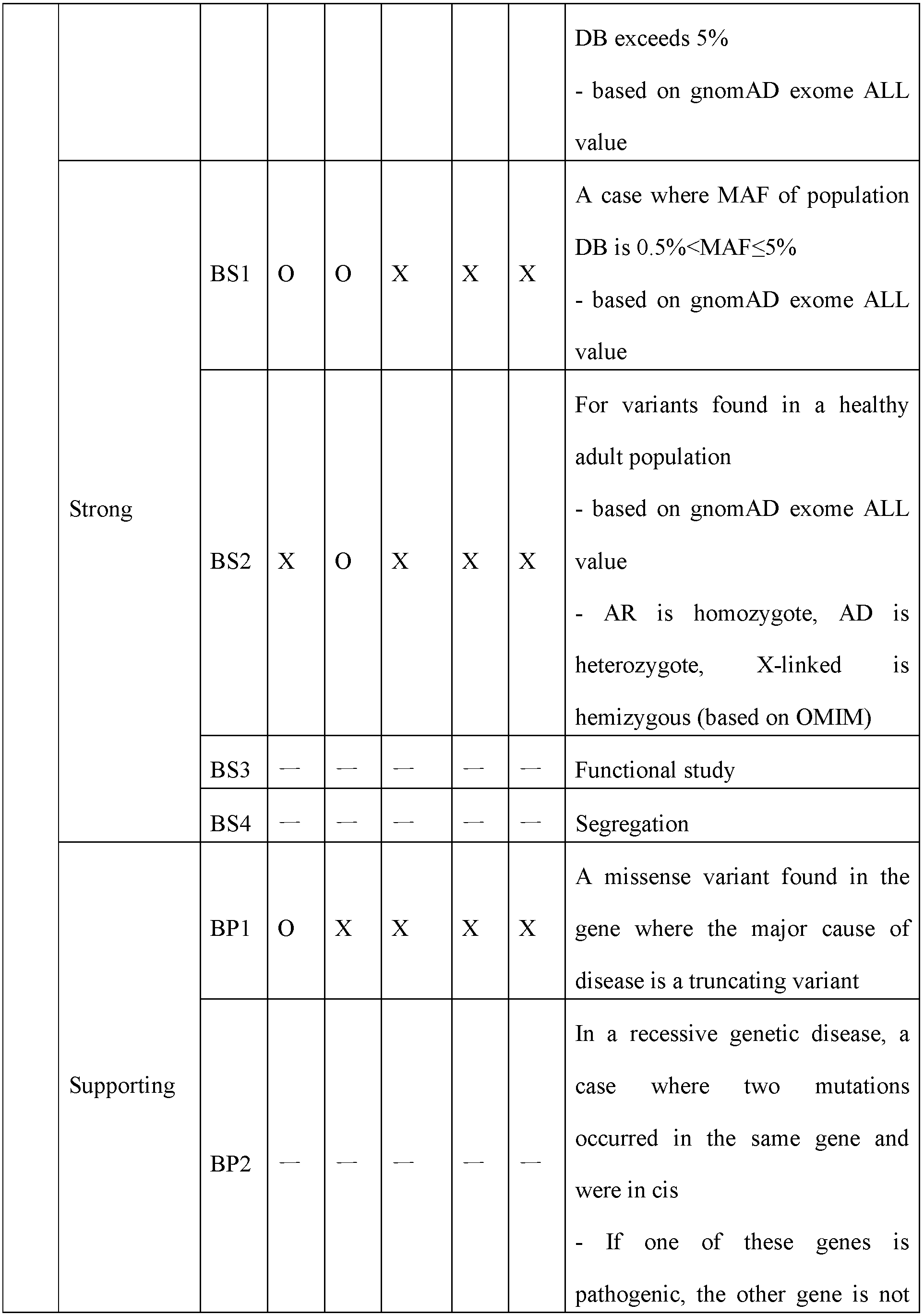

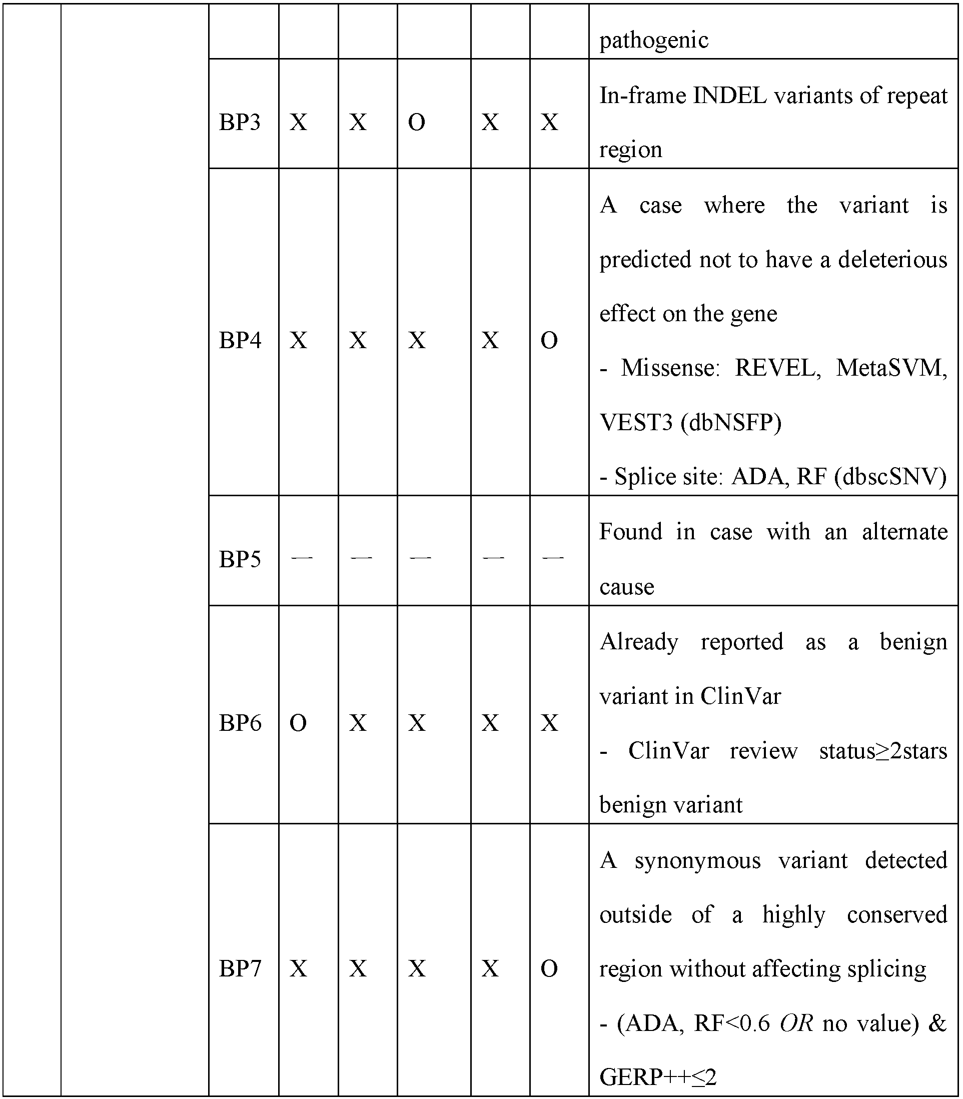

Table 7 below shows the pathogenicity determination logic tree of the present invention, obtained by applying the retrieved information to the method of classifying pathogenicity according to the ACMG guidelines.

**[Table 7]**

| **Classification** | **Conditions for determination** |
|---|---|
| **P (pathogenic)** | PVS=1 and PS≥1 *OR* |
| | PVS=1 and PM≥2 *OR* |
| | PVS=1 and PM≥1 and PP≥1 *OR* |
| | PVS=1 and PP≥2 *OR* |
| | PS≥2 *OR* |
| | PS=1 and PM≥3 *OR* |
| | PS=1 and PM≥2 and PP≥2 *OR* |
| | P S=1 and PM≥1 and PP≥4 |
| LP (likely pathogenic) | PVS=1 and PM=1 *OR* |
| | PS=1 and PM=1 *OR* |
| | PS=1 and PM=2 *OR* |
| | PS=1 and PP≥2 *OR* |
| | PM≥3 *OR* |
| | PM=2 and PP≥2 *OR* |
| | PM=1 and PP≥4 |
| LB (likely benign) | BS=1 and BP=1 *OR* |
| | BP≥2 |
| **B (Benign)** | BA=1 *OR* |
| | BS≥2 |
| **VUS (Uncertain Significance)** | A case where the above conditions are not met or if benign and pathogenic are in conflict OR if they are not classified as VUSp or VUSB |
| **VUSp** | Case of PM2 and PP3 and PM1 |
| **VUSb** | Having BP6 *OR* |
| | there are no ClinVar review status≥2 stars P, LP, and |
| | relevant gene's ClinVar review status≥2 stars P, LP variant Max. |
| | MAF < target variant MAF and |
| | not PP3 and not MAF>0.01% AND domain |

In Table 7 above, VUSp is the case of "PM2 and PP3 and PM1" and is classified as VUS in the ACMG guidelines, but in the logic tree (SAToK algorithm) of the present invention, VUSp is classified as a variant close to pathogenic, even though it is VUS. VUSb is "the case of having BP6" or "the case where there are no ClinVar review status≥2 stars P, LP and which is not relevant gene's ClinVar review status≥2 stars P, LP variant Max. MAF<target variant MAF and PP3 and not MAF>0.01% AND domain" and is classified as VUS in the ACMG guidelines, but in the logic tree (SAToK algorithm) of the present invention, VUSb is classified as a variant close to benign, even though it is VUS.

### Example 2. Verification of Logic Tree for Interpreting NGS Variant Data

The present inventors verified whether the logic tree for interpreting NGS variant data obtained in Example 1 can be reliably applied to practically interpret NGS variant data to determine pathogenicity.

Specifically, using a total of 52 patient samples (about 260 genes) tested with a gene panel for congenital metabolic abnormalities, a total of 3,373 non-overlapping variants to be analyzed were selected, and the selected variants were comparatively analyzed with the logic tree (SAToK algorithm) of the present invention and the control logic tree (InterVar algorithm). The InterVar algorithm used as the control was developed for the purpose of facilitating the interpretation of genetic variants based on nucleic acid sequencing, similar to the present invention, and is known in the art to which the present invention pertains (Am J Hum Genet. 2017 Feb 2;100(2):267-280). The logic tree of the present invention differs from the control logic tree in that it compares the information on genetic variants with clinical characteristic information, single-nucleotide polymorphism frequency information, repeat sequence information, protein domain information, and in-silico prediction information, whereas the control logic tree compares the information on genetic variants with clinical characteristic information, single-nucleotide polymorphism frequency information, and in-silico prediction information. In addition, there is a difference in that the logic tree of the present invention applies only highly reliable information (review status = 2) extracted from a database based on clinical characteristic information provided by the U.S. National Center for Biotechnology Information (NCBI), whereas the control logic tree applies all information without reliability verification.

As a result of the analysis, the level of pathogenicity was determined by each of the logic trees as shown in Table 8 below. Only for variants determined as "pathogenic (P)" or "likely pathogenic (LP)" by each of the logic tree of the present invention and the control logic tree, the accuracy of each of the logic tree of the present invention and the control logic tree was compared with that of the ClinVar algorithm (Nucleic Acids Res. 2016 Jan 4;44(D1):D862-8). The results are shown in FIG. 1.

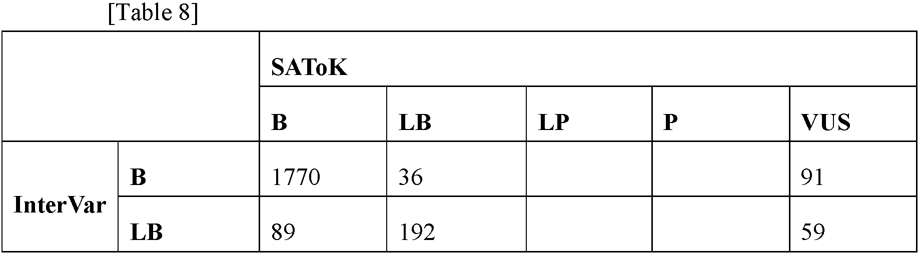

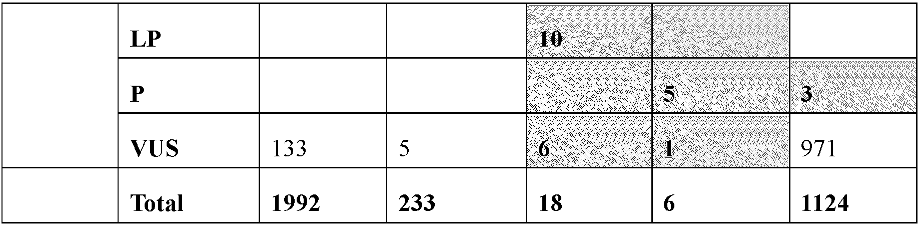

As shown in FIG. 1, it could be seen that the variants determined as "pathogenic (P)" or "likely pathogenic (LP)" by the logic tree of the present invention showed a first coincidence rate (case where the judgment is exactly the same) and second coincidence rate (when P or LP is recognized as the same judgment) of 50% and 80%, respectively, with the results determined by the ClinVar algorithm, but the control logic tree showed a first coincidence rate and second coincidence rate of 20% and 20%, respectively. This suggests that the logic tree of the present invention can be used quickly and accurately to classify the pathogenicity of genetic variants based on the ACMG guidelines.

Although the present disclosure has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The method of interpreting genetic variants according to the present invention provides a logic tree for interpreting NGS variant data, which can classify the pathogenicity of genetic variants based on the ACMG guidelines and determine the level of pathogenicity of the genetic variants, and thus it is expected to be widely used in the life sciences and medical health fields.

## Claims

1. A method for determining pathogenicity of genetic variants, comprising steps of:
(a) obtaining information on genetic variants from sequencing results;
(b) classifying pathogenicity of the genetic variants by comparing the information on the genetic variants with clinical characteristic information, single-nucleotide polymorphism frequency information, repeat sequence information, protein domain information, and in-silico prediction information, and
(c) determining a level of pathogenicity of the genetic variants.

2. The method of claim 1, wherein the clinical characteristic information, the single-nucleotide polymorphism frequency information, the repeat sequence information, the protein domain information, and the in-silico prediction information are extracted from public databases.

3. The method of claim 1, wherein step (b) of classifying pathogenicity of the genetic variants comprises classifying the pathogenicity as "very strong", "strong", "moderate", or "supporting" for pathogenic criteria, and classifying the pathogenicity as "stand-alone", "strong", or "supporting" for benign criteria.

4. The method of claim 3, wherein step (b) of classifying the pathogenicity of the genetic variants comprises classifying the pathogenicity as "very strong" stage 1, "strong" stage 1 to 4, "moderate" stage 1 to 4, or "supporting" stage 1 to 5 for pathogenic criteria, and classifying the pathogenicity as "stand-alone" stage 1, "strong" stage 1 to 4, or "supporting" stage 1 to 7 for benign criteria.

5. The method of claim 4, wherein the clinical characteristic information is applied to any one or more classes selected from the group consisting of very "strong" stage 1 for pathogenic criteria, "strong" stage 1 for pathogenic criteria, "moderate" stage 1 for pathogenic criteria, "moderate" stage 5 for pathogenic criteria, "supporting" stage 2 for pathogenic criteria, "strong" stage 1 for benign criteria, and "supporting" stage 6 for benign criteria.

6. The method of claim 4, wherein the single-nucleotide polymorphism frequency information is applied to any one or more classes selected from the group consisting of "moderate" stage 2 for pathogenic criteria, "stand-alone" stage 1 for benign criteria, "supporting" stage 1 for benign criteria, and "supporting" stage 2 for benign criteria.

7. The method of claim 4, wherein the repeat sequence information is applied to any one or more classes selected from the group consisting of "moderate" stage 4 for pathogenic criteria, and "supporting" stage 3 for benign criteria.

8. The method of claim 4, wherein the protein domain information is applied to the class of "moderate" stage 1 for pathogenic criteria.

9. The method of claim 4, wherein the in-silico prediction information is applied to any one or more classes selected from the group consisting of "supporting" stage 3 for pathogenic criteria, "supporting" stage 4 for benign criteria, and "supporting" stage 7 for benign criteria.

10. The method of claim 1, wherein step (c) of determining the level of pathogenicity comprises classifying the level of pathogenicity as "pathogenic", "likely pathogenic", "benign", "likely benign", or "uncertain significance".

11. The method of claim 10, wherein the "likely benign" is classified into uncertain significance, uncertain significance-pathogenic, and uncertain significance-benign.

12. An apparatus for determining pathogenicity of genetic variants, comprising:
(a) an input unit configured to input information on genetic variants obtained from sequencing results;
(b) a classification unit configured to classify pathogenicity of the genetic variants by comparing the information on genetic variants with clinical characteristic information, single-nucleotide polymorphism frequency information, repeat sequence information, protein domain information, and in-silico prediction information; and
(c) a determination unit configured to determine a level of pathogenicity of the genetic variants.

13. The apparatus of claim 12, wherein the clinical characteristic information, the single-nucleotide polymorphism frequency information, the repeat sequence information, the protein domain information, and the in-silico prediction information are extracted from public databases.

14. A method of predicting disease occurrence in a subject, comprising steps of:
(a) performing sequencing on a sample isolated from a subject of interest;
(b) determining pathogenicity of genetic variants according to the method of claim 1; and
(c) predicting disease occurrence in the subject based on the result of determining the pathogenicity.

15. A method of providing information for diagnosis of the cause of disease in a subject, comprising steps of:
(a) performing sequencing on a sample isolated from a subject of interest;
(b) determining pathogenicity of genetic variants according to the method of claim 1; and
(c) determining the cause of disease in the subject based on the result of determining the pathogenicity.
